# EUROPEAN PATENT APPLICATION

(11) **EP 4 134 075 A1**
(43) Date of publication of application: **15.02.2023**
(21) Application number: 21191707.5
(22) Date of filing: 17.08.2021
(51) Int. Cl.: A61K 31/122, A61K 31/22, A61K 31/426, A61K 31/4706, A61P 11/00, A61P 31/14

(54) **HEXAHYDRONAPHTHALEN-2-ONE DERIVATIVES FOR USE AGAINST SARS-COV-2**

(30) Priority: 09.08.2021 HU 2100293
(71) Applicant: Támogatott Kutatócsoportok Irodája, 1052 Budapest (HU); Eötvös Loránd Tudományegyetem, 1053 Budapest (HU); Nemzeti Népegészségügyi Központ, 1097 Budapest (HU)
(72) Inventor: Kovács M., Gábor, 1014 Budapest (HU); Boldizsár, Imre, 1082 Budapest (HU); B sze, Szilvia Erika, 1124 Budapest (HU); Horváti, Kata, 1094 Budapest (HU); Kis, Zoltán, 4400 Nyíregyháza (HU); Pályi, Bernadett, 1026 Budapest (HU)
(74) Representative: Danubia Patent & Law Office LLC

(57) **Abstract**

The invention relates to hexahydronaphthalen-2-one derivatives, such as petasol, petasin, isopetasol and isopetasin, for use in the treatment of viral infections. In particular compounds extracted from Darksidea spp. are provided for use in the treatment of SARS-CoV-2 infection.

## Description

### FIELD OF THE INVENTION

The invention relates to compounds for the treatment of viral infections. In particular compounds extracted from Darksidea spp. are provided for use in the treatment of SARS-CoV-2 infection.

### BACKGROUND OF THE INVENTION

The Severe acute respiratory syndrome-related coronavirus 2 (SARS-CoV-2), which caused the COVID-19 pandemic outbreak, belongs to the Coronaviridae family. Coronaviruses are enveloped single-stranded positive-sense RNA viruses and are recognized as a continuous zoonotic threat with the ability to cause respiratory, enteric, hepatic, or neurological diseases with highly variable severity in humans. Recently, three zoonotic coronaviruses caused widespread outbreaks: SARS-CoV in 2002-2003 resulted approximately 800 deaths in 30 countries [Drosten, C., et al., Identification of a novel coronavirus in patients with severe acute respiratory syndrome. N Engl J Med, 2003. 348: 1967-76.; Peiris, J.S., Y. Guan, and K.Y. Yuen, Severe acute respiratory syndrome. Nat Med, 2004. 10: S88-97.]; Middle East respiratory syndrome MERS-CoV in 2012 leading to 858 known deaths in 27 countries [Zaki, A.M., et al., Isolation of a novel coronavirus from a man with pneumonia in Saudi Arabia. N Engl J Med, 2012. 367: 1814-20.; Assiri, A., et al., Hospital outbreak of Middle East respiratory syndrome coronavirus. N Engl J Med, 2013. 369: 407-16.; de Wit, E., et al., SARS and MERS: recent insights into emerging coronaviruses. Nat Rev Microbiol, 2016. 14: 523-34.] and the current SARS-CoV-2 [Holwerda, M., et al., Identification of an antiviral compound from the pandemic response box that efficiently inhibits SARS-CoV-2 infection in vitro. Microorganisms, 2020. 8: 1872.]. As of 25 April 2021, a total of 146,054,107 cases of COVID-19 have been confirmed including 3,092, 410 deaths.

Currently, there are no approved therapies for the treatment of COVID-19 except for an emergency use authorization for Remdesivir (FDA EUA: 29 April 2020). Based on the results of previous trials against similar coronaviruses such as SARS-CoV and MERS-CoV, many treatment options have been suggested and implicated in new clinical trials to study the effectiveness against SARS-CoV-2. However, results are controversial and we still lack effective antiviral compounds against SARS-CoV-2 caused respiratory failure from acute respiratory distress syndrome, which is the leading cause of mortality in COVID-19 patients.

The driving force behind many of the early therapies suggested for COVID-19 were the repurposing of antiviral drugs. Remdesivir, chloroquine / hydroxychloroquine, favipiravir and lopinavir / ritonavir were among the first set of the trials with antivirals. Later on, emerging evidences of hyperinflammation and cytokine release syndrome highlighted the potential use of immunmodulating drugs, such as corticosteroids and interleukin inhibitors [Rebold, N., et al., COVID-19: Before the Fall, An evidence-based narrative review of treatment options. Infect Dis Ther, 2021. 10: 93-113.]. The road to effective COVID-19 treatment continuous with the discovery of new compounds and for that, systematic in vitro search is urgent and indispensable.

As to the treatment of viral infections, two formally approved drugs could be regarded as natural product (i.e., the lignan podofilox and the flavonoid sinecatechin) [De Clercq, E. and Li, G., Approved antiviral drugs over the past 50 years. Clin Microbiol Rev, 2016. 29: 695-747.]. Furthermore, more than 100 plant species were confirmed to express antiviral properties [Martinez, J.P., et al., Antiviral drug discovery: broad-spectrum drugs from nature. Nat Prod Rep, 2015. 32: 29-48.].

*Darksidea* belong to the root-colonising fungi, dark septate endophytes (DSE) [Knapp, D.G., et al., Dark septate endophytic pleosporalean genera from semiarid areas. Persoonia, 2015. 35: 87-100. ] To date, no medicinal use of *Darksidea* has been reported.

Petasol (3S,4aR,5R,6R)-6-hydroxy-4a,5-dimethyl-3-prop-1-en-2-yl-3,4,5,6,7,8-hexahydronaphthalen-2-one) and its isomers, isopetasol (4aR,5R,6R)-6-hydroxy-4a,5-dimethyl-3-propan-2-ylidene-5,6,7,8-tetrahydro-4H-naphthalen-2-one) and neopetasol (3R,4aR,5R,6R)-6-hydroxy-4a,5-dimethyl-3-prop-1-en-2-yl-3,4,5,6,7,8-hexahydronaphthalen-2-one) are eremophilane sesquiterpenoid compounds produced by e.g. *Petasites formosanus* and *Penicillium sp.,* which have been tested for the treatment of e.g. malaria, cancer and HIV.

### SUMMARY OF THE INVENTION

A compound for use in the prevention and/or treatment of a viral infection is provided. The virus is an RNA virus, preferably selected from coronaviruses, ebolaviruses, mammarenaviruses, in particular Lassa mammarenavirus, henipaviruses, in particular Nipah henipavirus and orthonairoviruses, in particular Crimean-Congo hemorrhagic fever orthonairovirus, preferably the virus is a coronavirus, preferably a SARS coronavirus, highly preferably SARS-CoV-2.

A compound of general formula (I) for use in the treatment of a coronavirus infection, 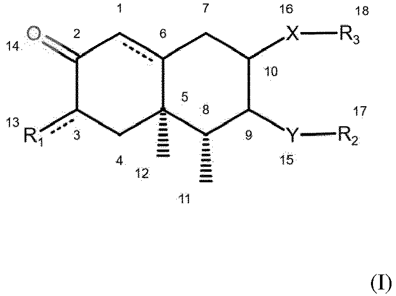 wherein in general formula (I)
the bond between carbon 1 and 6 is either a single bond or a double bond,
the bond between carbon 13 of R₁ and carbon 3 is either a single bond or a double bond, wherein R₁ is
   isopropyl, if the bond between R₁ and carbon 3 is a double bond, or
   isopropenyl, if the bond between R₁ and carbon 3 is a single bond,
X is O or nothing
R₂ is H or R₂ has the following formula (i) 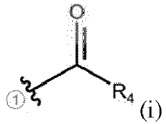
   wherein in formula (i) R₄ is a C₁-C₈ alkyl or a C₁-C₈ alkenyl or a C₁-C₇ thioalkyl or a C₁-C₇ thioether, or a C₁-C₈ alkyl or a C₁-C₈ alkenyl substituted with a single -SH group, wherein said C₁-C₈ alkenyl is a straight or branched chain alkenyl;
   or
   X is O or nothing
R₃ is H or R₃ has the following formula (i) 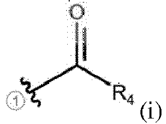
   wherein in formula (i) R₄ is a C₁-C₈ alkyl or a C₁-C₈ alkenyl or a C₁-C₇ thioalkyl or a C₁-C₇ thioether, or a C₁-C₈ alkyl or a C₁-C₈ alkenyl substituted with a single -SH group, wherein said C₁-C₈ alkenyl is a straight or branched chain alkenyl;
   or
   Y is O or nothing
wherein one of X and Y is O, and if X is nothing, then R₃ is H and if Y is nothing then R₂ is H.

Preferably the compound has general formula (II) 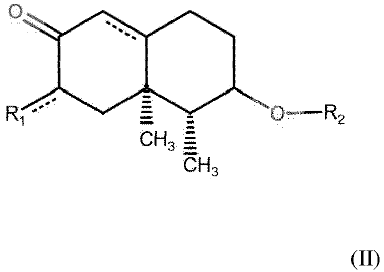 wherein in general formula (II)
the bond between carbon 1 and 6 is either a single bond or a double bond, preferably a double bond,
the bond between carbon 13 of R₁ and carbon 3 is either a single bond or a double bond, wherein R₁ is
   isopropyl, if the bond between R₁ and carbon 3 is a double bond, or
   isopropenyl, if the bond between R₁ and carbon 3 is a single bond,
R₂ is H or R₂ has the following formula (i) 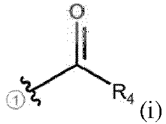 wherein in formula (i) R₄ is a C₁-C₈ alkyl or a C₁-C₈ alkenyl or a C₁-C₇ thioalkyl or a C₁-C₇ thioether, or a C₁-C₈ alkyl or a C₁-C₈ alkenyl substituted with a single -SH group, wherein said C₁-C₈ alkenyl is a straight or branched chain alkenyl.

Preferably the compound has general formula (III) 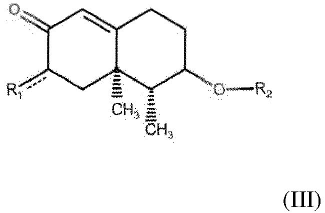 wherein wherein R₁ is and R₂ are as defined above.

Preferably the compound is selected from the group consisting of compounds having general formulae IV.1, IV.2, IV.3,

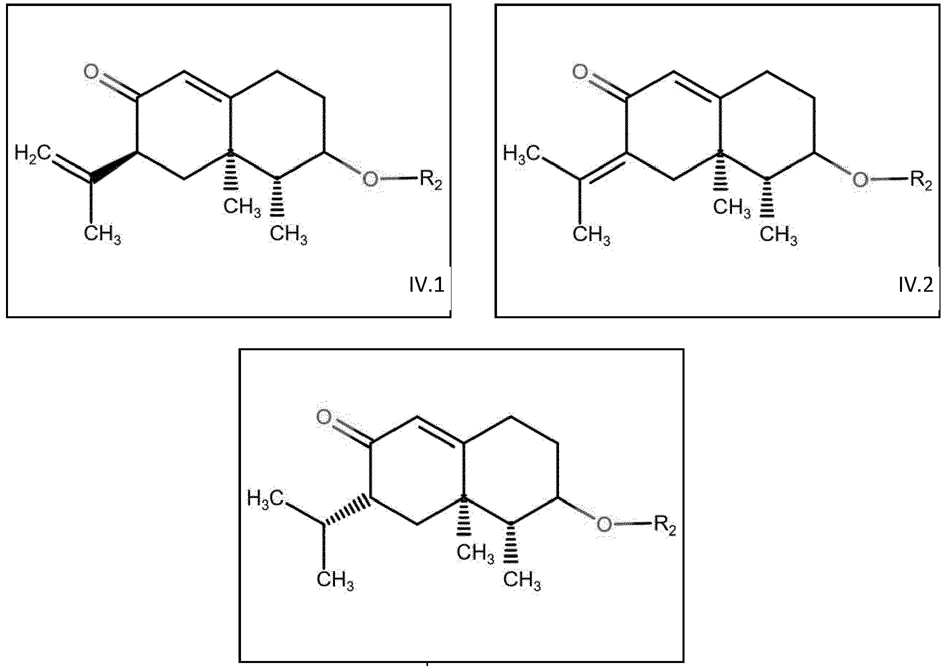

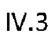

preferably general formulae IV.1, IV.2,
wherein R₂ is as defined above.

Preferably
R₂ is selected from the group consisting of

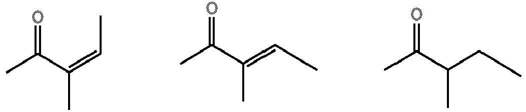

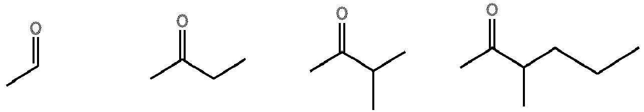

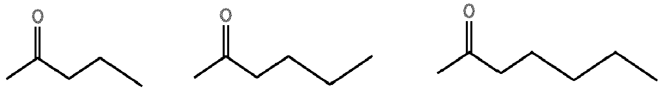

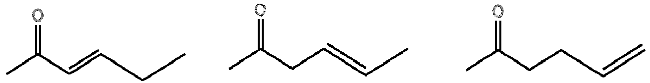

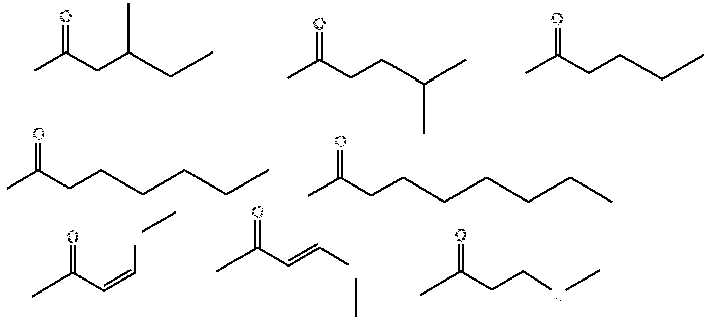

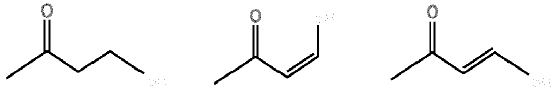

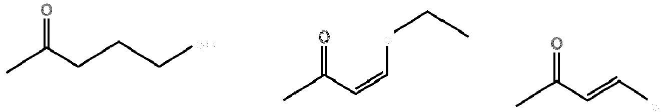

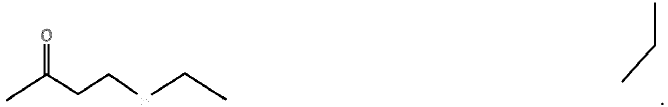

Preferably when the bond between C3 and R1 is a single bond, the compound is the S isomer. Preferably the compound is selected from the group consisting of petasol and esters thereof, isopetasol and esters thereof.

Preferably the compound is selected from the group consisting of petasol, petasin, S-petasin, isopetasol, isopetasin and S-isopetasin.

Preferably the compound is selected from the group consisting of petasol and isopetasol.

Preferably the compound is selected from the group consisting of petasol, petasin and S-petasin. Preferably the coronavirus is SARS-CoV-2.

### BRIEF DESCRIPTION OF THE DRAWINGS

Figure 1. Representative microscopic image of non-infected Vero-E6 monolayer (A) and CPE after 48 hours of infection with SARS-CoV-2 (B). (Nikon Eclipse, TS2R)
Figure 2. HPLC-UV (λ=250 nm) chromatogram of the methyl alcohol extract obtained from the in vitro culture of Darksidea alpha. Peaks at retentions 7.3, 7.6 and 8.5 min correspond to the compounds DA-1 (petasol), DA-2 (isopetasol) and DA-3 (neopetasol), respectively.
Figure 3. HR-MS spectra of the compounds DA-1 (petasol) (A), DA-2 (isopetasol) (B) and DA-3 (neopetasol) (C), obtained from the HPLC-MS chromatogram of Darkside alpha extract.
Figure 4. UV spectra of petasol (A) isopetasol (B) and neopetasol (C) obtained from the HPLC-UV chromatogram of Darksidea alpha extract.
Figure 5. Chemical structure of petasol (A), isopetasol (B) and neopetasol (C).
Figure 6. Validation of the antiviral assay.
Figure 7. Cytotoxicity of the compounds. VeroE6 cells were treated with the compounds at 0.8-100 µM concentration range for 48 hrs. Cell viability was measured then by Alamar Blue assay.
Figure 8. Antiviral effect of the compounds against SARS-CoV-2. VeroE6 cells were infected with SARS-CoV-2 and incubated with the compounds for 48 hrs. Vero-E6 cell monolayer plates were inoculated with the virus-containing supernatants and after 5 days, cells were fixed and stained with crystal violet, then TCID50/mL values were calculated.
Figure 9. Antiviral effect of petasol and its two isomers.
Figure 10. Effect of petsol, nitrazoxanide and chloroquine on viral copy number.

### DETAILED DESCRIPTION OF THE INVENTION

The term "tioalkyl" as used herein refers to a saturated (i.e., S-alkyl) or unsaturated (i.e., S-alkenyl and S-alkynyl) group attached to the parent molecular moiety through a sulfur atom. In certain embodiments, the alkyl group contains 1-20 aliphatic carbon atoms. In certain other embodiments, the alkyl group contains 1-10 aliphatic carbon atoms. In yet other embodiments, the alkyl, alkenyl, and alkynyl groups employed in the invention contain 1-8 aliphatic carbon atoms. In still other embodiments, the alkyl group contains 1-6 aliphatic carbon atoms. In yet other embodiments, the alkyl group contains 1-4 aliphatic carbon atoms. Examples of thioalkyl include, but are not limited to, methylthio, ethylthio, propylthio, isopropylthio, n-butylthio, and the like.

Petasol is an eremophilane sesquiterpenoid compound, having the structure (A): 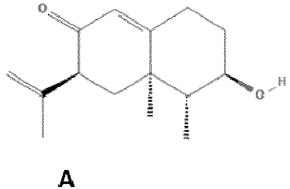 and isomers isopetasol, having the structure (B): 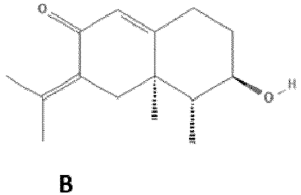 and neopetasol, having the structure (C):

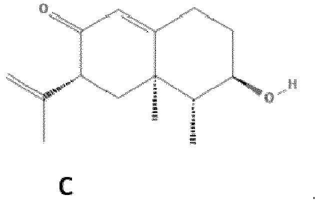

Petasol may be synthesized by the method described in Neuenschwander, M., Neuenschwander, A. and Steinegger, E. (1979), Struktur der Sesquiterpene von Petasites hybridus (L.) G. M. et SCH.: Neopetasol-Abkömmlinge. HCA, 62: 627-634. https://doi.org/10.1002/hlca.19790620229, while the synthesis of isopetasol was reported by Bohlmann and Otto (Natürlich vorkommende Terpen-Derivate, 3831) Synthese des Isopetasols. Liebigs Ann. Chem., 1982: 186-190. https://doi.org/10.1002/jlac.198219820119) and Neuenschwander et al (supra), Yamakawa et al. (Chem. Pharm. Bull., 1979, 27, 331-340. b) Torii et al. (Bull. Chem. Soc. Jpn., 1979, 52, 861-866). More reference to the synthesis of petasol and isopetasol are found in Pirrung et al. (The Total Synthesis of Natural Products: Bicyclic and Tricyclic Sesquiterpenes. John Wiley & Sons, 22. Sept. 2009); Burow, Kenneth Wayne Jr., "Approaches to the synthesis of the petasin sesquiterpenes" (1973). Retrospective Theses and Dissertations. 6138. https://lib.dr.iastate.edu/rtd/6138); DE4447594A1. Petasol and its isomers may be isolated from a number of sources with methods well-known in the art. See e.g. Lin et al. (Eremophilanes from Petasites formosanus Kitamura. Chem. Pharm. Bull. 46(11) 1807-1809 (1998)) and Debrunner and Neuenschwander (Sesquiterpenes of Petasites hybridus ( L.) G.M. et Sch.: influence of locations and seasons on sesquiterpene distribution. Pharmaceutics Acta Helvetiae 70 (1995) 315-323) from *Petasites formosanus;* Jayasuriya et al. Identification of Diverse Microbial Metabolites as potent Inhibitors of HIV-1 Tat Transactivation. Chem Biodivers. 2005 Jan;2(1):112-22. doi: 10.1002/cbdv.200490162.) from *Penicillium sp.* Isolation of petasol and its isomers from *Darksidea alpha* is described in the Examples.

Identification of the isolated compounds can be performed by conventional methods, such as HPLC and NMR. An example of such methods is given in the Examples.

Petasin (CAS: 26577-85-5) is an ester of angelic acid and petasol, having the structure (D) 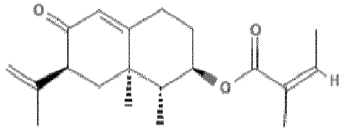 and the isomers isopetasin (CAS: 469-26-1), having the structure (E) 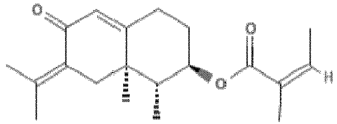 and neopetasin (CAS: 70387-53-0), having the structure (F)

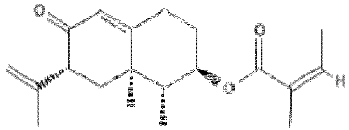

Synthesis of petasin and isopetasin is described in e.g. Burns and Taylor Synthetic Approaches to Enantiomerically Enriched 4-Hydroxycyclohex-2-en-1-one - A Key Chiral Building Block in Complex Natural Product Synthesis; Synthesis 2011. No. 5, pp0681-0707 and in DE4447594. Extraction of petasin is described e.g. in EP2485718, EP0281656, CH690355 and KR20150047814. S-petasin (CAS:70238-51-6) is a methylsulfanyl derivative of petasin, having the structure (G) 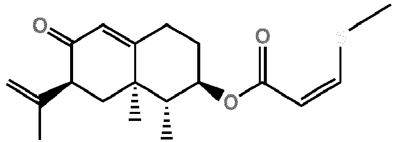 and the isomers S-isopetasin with the structure 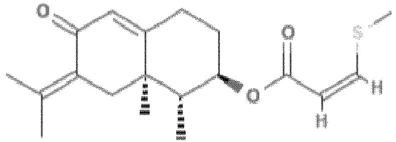 and S-neopetasin (CAS: 87984-58-5) with the structure

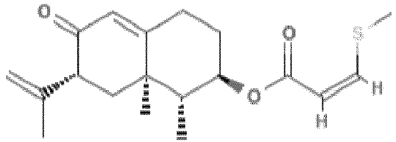

A method for producing an extract containing S-petasin and its isomers from cranberry is described in WO2009149288, from Petasites hybridus in WO2011042469 and by Aebi et al.. Inhaltsstoffe von Petasites hybridus (L.) Fl. Wett. Pharmaceutica acta Helvetiae. 1995;29:277-279 (containing petasin, isopetasin, S-petasin and S-isopetasin), Lin Y-L, Mei C-H, Huang S-L, Kuo Y-H. Four new sesquiterpenes from Petasites formosanus. Journal of Natural Products. 1998;61(7):887-890.

Up to date, six species in the Darksidea genus have been characterized: D. alpha, beta, gamma, delta, epsilon and zeta [Knapp, D.G., et al., Dark septate endophytic pleosporalean genera from semiarid areas. Persoonia, 2015. 35: 87-100. ]. The genus has a worldwide distribution and has been detected in several different habitats, mainly grassland. The biggest amount of petasol (together with isopetasol and neopetasol) produced was detected in D. alpha, however, isolates from other species also produced these compounds.

The production of petasol and/or any of the compounds described herein by a plant or fungus species may be detected by well known methods (an example is given in the Examples section) and the extraction of petasol and/or any of the compounds for use according to the invention may be extracted from plants or fungus by known methods (see references above and the Examples). Petasol and isopetasol may be isolated from any of the Darksidea species by the method described in the Examples, comprising:
(a) culturing an isolate of a Darksidea sp. under suitable conditions (such as a medium allowing growth of the culture (e.g. Potato agar dextrose), a temperature allowing growth of the culture (e.g. room temperature) and light conditions allowing growth of the culture (e.g. dark)) allowing the production of petasol by the cells,
(b) obtaining an extract of the culture (either the complete culture comprising medium and fungi or the fungi alone or the medium alone) by a suitable method (e.g. by alcoholic extraction),
(c) isolating the desired compound by suitable means (e.g. preparative HPLC).

Culturing may be performed in a bioreactor.

Accordingly, a method for the production of petasol, isopetasol and/or neopetasol is provided, comprising steps (a)-(b) and optionally (c) defined above.

In another aspect, use of a Darksidea sp., highly preferably Darksidea alpha, for the production of petasol, isopetasol and/or neopetasol is provided, comprising steps (a)-(b) and optionally (c) defined above.

A pharmaceutical composition comprising a compound according to formula (I) and a pharmaceutically acceptable excipient for use in the treatment of an RNA virus infection is also provided. Preferably the pharmaceutical composition is suitable for oral administration, e.g. is in the form of a tablet, powder, capsule. In other embodiments the pharmaceutical composition is suitable for parenteral administration, e.g. intravenous administration.

In another aspect, an extract comprising a compound according to formula (I) derived from a plant or a fungus is provided for use in the treatment of an RNA virus infection. Preferably the plant belongs to a Petasites sp. or the fungus belongs to a Darksidea sp., preferably to Darksidea alpha. Preferably the extract is enriched in the compound. Preferably the compound is selected from the group consisting of petasol and esters thereof, isopetasol and esters thereof. Preferably the compound is selected from the group consisting of petasol, petasin, S-petasin, isopetasol, isopetasin and S-isopetasin. Preferably the compound is selected from the group consisting of petasol and isopetasol. Preferably the compound is selected from the group consisting of petasol, petasin and S-petasin.

In another aspects the compound for use is a prodrug of petasol or isopetasol, which may be converted into petasol or isopetasol, respectively, upon administration to a subject in need thereof and a pharmaceutical composition comprising the prodrug and a pharmaceutically acceptable excipient for use in the treatment of a coronavirus infection is provided.

Preferably the RNA virus is selected from enveloped, positive-strand RNA viruses. Preferably the RNA virus is selected from from coronaviruses, ebolaviruses, mammarenaviruses, in particular Lassa mammarenavirus, henipaviruses, in particular Nipah henipavirus and orthonairoviruses, in particular Crimean-Congo hemorrhagic fever orthonairovirus, preferably the virus is a SARS coronavirus, highly preferably SARS-CoV-2. In another preferred embodiment the RNA virus is selected from coronaviruses (family *Coronaviridae,* genera *Coronavirus: Alphacoronavirus, Betacoronavirus, Gammacoronavirus, Deltacoronavirus),* preferably respiratory coronaviruses, e.g. bovine respiratory coronavirus, canine respiratory coronavirus, feline respiratory coronavirus, infectious bronchitis viruses, and coronaviruses causing mainly respiratory, enteric, hepatic and neurological symptomps, such as rabbit coronavirus, feline enteric coronavirus (FECV), feline infectious peritonitis virus (FIPV), transmissible gastroenteritis virus (TGEV), porcine epidemic diarrhoea virus (PEDV), and porcine deltacoronavirus (PDCoV). Preferably the subject to be treated is a mammal or an avian subject, e.g. a human, a companion animal (such as a dog, cat or rabbit), a domesticated or farm animal, such as cattle, swine, sheep, fowl or a wild animal, such as bats, big cats, hares or african wild dogs.

### EXAMPLES

### Fungal isolates

The fungal isolates from which DA-1 (petasol), DA-2 (isopetasol) and DA-3 (neopetasol) were isolated, represent the widespread root endophytic fungi Darksidea alpha (Ascomycota, Pleosporales). The isolates are in the fungal root endophyte strain collection at the Mycological Research Group (Department of Plant Anatomy, Institute of Biology, Eötvös Loránd University). Albeit several isolates were screened and they produced the compounds, the most efficient producers were the isolates DSE7/1, DSE7/15 and DSE7/20 (Table 1-2). All those isolates belong to our collection based on which we described the genus and species [Knapp, D.G., et al., Dark septate endophytic pleosporalean genera from semiarid areas. Persoonia, 2015. 35: 87-100.]. We used phylogenetic analyses of sequences of seven nuclear DNA loci for the species identification of the isolates, nrDNA ITS: internal transcribed spacer regions of the nrDNA and intervening 5.8S nrDNA; LSU: partial 28S large subunit of the nrRNA gene; SSU: partial 18S small subunit of the nrRNA gene; ACT: partial actin gene; TUB: partial beta-tubulin gene; CAL: partial calmodulin gene; TEF: partial translation elongation factor 1-alpha gene. All of the strains were isolated from surface sterilized healthy root segments of healthy-looking host plants [Knapp, D.G., et al., Dark septate endophytic pleosporalean genera from semiarid areas. Persoonia, 2015. 35: 87-100.; Knapp, D.G., A. Pintye, and G.M. Kovacs, The dark side is not fastidious - dark septate endophytic fungi of native and invasive plants of semiarid sandy areas. PLoS One, 2012. 7: e32570.].

**Tables 1-2. The host, geographic origin and GenBank accession numbers of DNA sequences of the three Darksidea alpha isolates, which produced the highest amounts of the three compounds.**

| **Isolate** | **Host** | **Location** | | **ITS** | **LSU** |
|---|---|---|---|---|---|
| **DSE7/1** | *Bromus tectorum* | Fülöpháza, Hungary | | KP183966 | KP184005 |
| **DSE7/15** | *Ailanthus altissima* | Tatárszentgyörgy, Hungary | | KP183970 | KP184037 |
| **DSE7/20** | *Festuca vaginata* | Fülöpháza, Hungary | | KP183994 | KP184015 |
| | | | | | |

| **Isolate** | **SSU** | **ACT** | **TUB** | **CAL** | **TEF** |
|---|---|---|---|---|---|
| **DSE7/1** | KP184005 | KP184093 | KP184192 | KP184122 | KP184160 |
| **DSE7/15** | KP184055 | KP184086 | KP184205 | KP184124 | KP184163 |
| **DSE7/20** | KP184066 | KP184084 | KP184210 | KP184128 | KP184164 |

### Preparation of fungal extracts for analysis and isolation

Isolates of Darksidea alpha were grown in Petri dishes (60 mm) on Potato dextrose agar (PDA) medium (VWR, Hungary) at room temperature in dark for 30 days. Complete in vitro cultures containing the medium and fungal mycelium were lyophilized and pulverized. These powders were extracted three times. First, they were extracted with 5 mL of methyl alcohol held under reflux at boiling point for 30 min. Thereafter, the insoluble, centrifuged material was extracted for a second time and a third time, as before. The combined supernatants were dried by a rotary vacuum evaporator at 40 °C. The dried extracts were dissolved in 5 mL of methanol. These solutions were used 1) subsequent to their dilution with methyl alcohol to identify the compounds by analytical highperformance liquid chromatography (HPLC) hyphenated with ultraviolet (UV) and high-resolution mass spectrometry (HR-MS) detections and 2) to isolate compounds by preparative HPLC.

### Analytical HPLC hyphenated with UV and HR-MS detections

A Dionex Ultimate 3000 UHPLC system (3000RS diode array detector (DAD), TCC-3000RS column thermostat, HPG-3400RS pump, SRD-3400 solvent rack degasser, WPS-3000TRS autosampler), hyphenated with a Orbitrap Q Exactive Focus Mass Spectrometer equipped with electrospray ionization (ESI) (Thermo Fischer Scientific, Waltham, MA, USA) was used for chromatographic separation and high resolution mass spectral analysis. The HPLC separations were performed on a Kinetex C18 column (75 × 3 mm; 2.6 µm) (Phenomenex, USA). Eluents: eluent A, 0.1% v/v formic acid, eluent B, acetonitrile:0.1% v/v formic acid (80:20, v/v). Linear gradient: 0.0 min, 20% B; 10.0 min, 70% B; flow rate: 0.3 mL/min; column temperature: 25 °C; injected volume: 1.0-5.0 µL. The ESI source was operated in positive ionization mode and operation parameters were optimized automatically using the built-in software. The working parameters were as follows: spray voltage, 3500 V (+); capillary temperature 256 °C; sheath-, auxiliary- and spare-gases (N2): 47.50, 11.25 and 2.25 arbitrary units, respectively. The resolution of the full scan was of 70,000 and the scanning range was between 100-1000 m/z units. DAD spectra were recorded between 230 and 600 nm.

### Preparative HPLC

A Pharmacia LKB HPLC (Uppsala, Sweden) system (2248 pumps, VWM 2141 UV detector) was connected to a preparative HPLC column: Gemini, 5 µm, C6-Phenyl, 100 × 21.2 mm (Phenomenex, USA). The eluents were the same as described above. Linear gradient: 0.0 min, 10% B; 20.0 min, 70% B; flow rate: 5.0 mL/min; column temperature: ambient; injected volume: 500 µL.

### Nuclear magnetic resonance (NMR) spectroscopy

NMR spectra of the isolated compounds were recorded in chloroform-d at 25 °C on a Varian DDR spectrometer (599.9 MHz for 1H and 150.9 MHz for 13C) equipped with a dual 5 mm inverse detection gradient (IDPFG) probe-head. Chemical shifts were referenced relative to the appropriate solvent resonances.

### Compounds

Compounds to be tested were dissolved in DMSO at 20 mM concentration and stored at -20 oC. Stock solutions were diluted with DMEM medium to result 100 µM concentration. The DMSO content was 0.5% in the highest concentration containing wells.

### Virus and cells

All infection experiments were performed under Biosafety Level 3 (BSL-3) conditions at the National Biosafety Laboratory, National Public Health Center (Budapest, Hungary).

Vero-E6 cell line was obtained from the European Collection of Authenticated Cell Cultures (ECACC 85020206) and maintained in DMEM high-glucose (4,5 g/L) medium (Lonza) containing 10% FBS (Gibco) and supplemented with2 mM of L-glutamine (Lonza)1 mM sodium pyruvate (Merck), CellCultureGuard (PanReacApplichem) at 37oC in a humidified atmosphere of 5% CO2.

The virus isolated on Vero E6 cells from an upper respiratory specimen of a COVID-19 patient in March, 2019 and passaged three times. The Vero-E6 cells was maintained in the previously described DMEM medium supplemented with 5% FBS and CellCultureGuard (PanReacApplichem,). The viral titer was determined by 50% tissue culture infective dose: TCID50 / mL= 106.

### Cytotoxicity assay

In vitro cytotoxicity testing of the compounds was performed by the Alamar Blue cell viability assay. Briefly, Vero-E6 cells were plated in a 96-well flat bottom tissue culture plates in 10% FBS containing DMEM media (5000 cells / 100 µL) one day prior to experiment. Before the test, 50 µL supernatant was removed and replaced with 50 µL DMEM serum free (SFM) medium. Compounds were serially diluted with SFM DMEM medium and added to the cells (final concentration range: 0.02 - 100 µM, final FBS concentration: 2.5%). Two days after, 20 µL Alamar Blue (resazurin sodium salt, Merck) solution (0.15 mg/mL in PBS, filtered 0,45 µ M Merck) was added to each well and after 3-3.5 hrs of incubation the fluorescence was measured at λEx = 530/30 and at λEM = 610/10 nm using a Synergy H4 multi-mode microplate reader (BioTek). All measurements were performed in quadruplets and the mean IC50 values together with SEM were represented on the graphs.

### Virus titration and antiviral effect of the compounds

Vero E6 cells were plated in 96-well flat bottom tissue culture plates (TPP) in 10% FBS containing DMEM medium (5000 cells / 100 µL) 24 hrs prior to experiment. After removing the supernatant, Vero E6 cells were treated for 1 hour with the serial dilution of the compounds in DMEM media. As negative control DMEM containing 0.5% DMSO medium was applied.

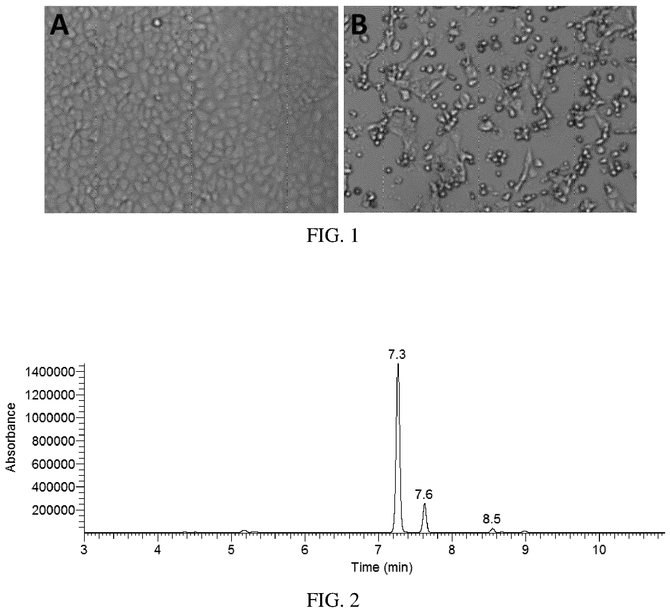

After treatment, Vero E6 cells were infected with SARS-CoV-2 virus at a multiplicity of infection (MOI) of 0.05, then cells were incubated with the viruses for further 1.5 hrs. After the removal of the supernatant from the wells, infected cells were incubated with 200 µL serially diluted compound solutions in DMEM containing 5% FBS. The inoculated cultures were growing in a humidified 37°C incubator in an atmosphere of 5% CO2 and observed for cytopathic effect (CPE) daily. Microscopic images were taken 2 days after infection (Fig. 1). When CPEs were observed, typically 48 hrs after infection, 50 µL of virus-containing supernatants were transferred to 96-well plates to determine the infective titer and kept in -80°C until further use.

To estimate the effect of the treatment by infective titration we determined the TCID50/mL values from each dilutions from the supernatant. The supernatant were diluted in 10-fold serial dilution in a dilution plate and were added to a Vero-E6 cell monolayers in 96-well cell cultureplate. The plates were incubated for 5days, the supernatant were removed and the plates were inactivated by 10% formaldehyde in PBS solution for 30 minutes, washed with tap water and stained with 0.5% crystal violet in ethanol for 15 minutes at room temperature. Clear CPE were visualized and the viral titers were measured by determining the TCID50/mL using TCID50 calculator v2.1 [Binder M. TCID50 Calculator (v2.1-20-01-2017_MB) [(accessed on 10 June 2020)]; available online: https://www.klinikum.uni-heidelberg.de/fileadmin/inst_hygiene/molekulare_virologie/Downloads/TCID50_calculator_v2_17-01-20_MB.xlsx.]. Experiments were done in quadruplets and repeated at least three times. The assay was validated by a negative and a positive control (medium treated virus control and chloroquine treated control).

### Viral copy number by RT-qPCR

From the supernatant viral RNA was extracted using the Perkin Elmer Chemagic Viral RNA/DNA Kit (PerkinElmer, The Netherlands) according to the manufacturer's instructions on a Perkin Elmer Chemagic automated extraction machine. Real-time RT-qPCR was conducted using SARS-CoV-2 RT-qPCR kit (PerkinElmer, The Netherlands) on Roche LightCycler 480 with automated second derivative evaluation method.

### RESULTS

### Isolation and Identification of the compounds

The DNA sequence information based on which the Darkside alpha as species was described [Knapp, D.G., et al., Dark septate endophytic pleosporalean genera from semiarid areas. Persoonia, 2015. 35: 87-100.] make the unambiguous identification of the isolates possible. Although we worked from our strain collection, whenever confirmation of the identity of the isolates was needed, the nrDNA ITS region was amplified and sequenced.

The culture extracts of the fungus Darksidea alpha contained three main compounds (Fig. 2, peaks at 7.3, 7.6 and 8.5 min retention times).

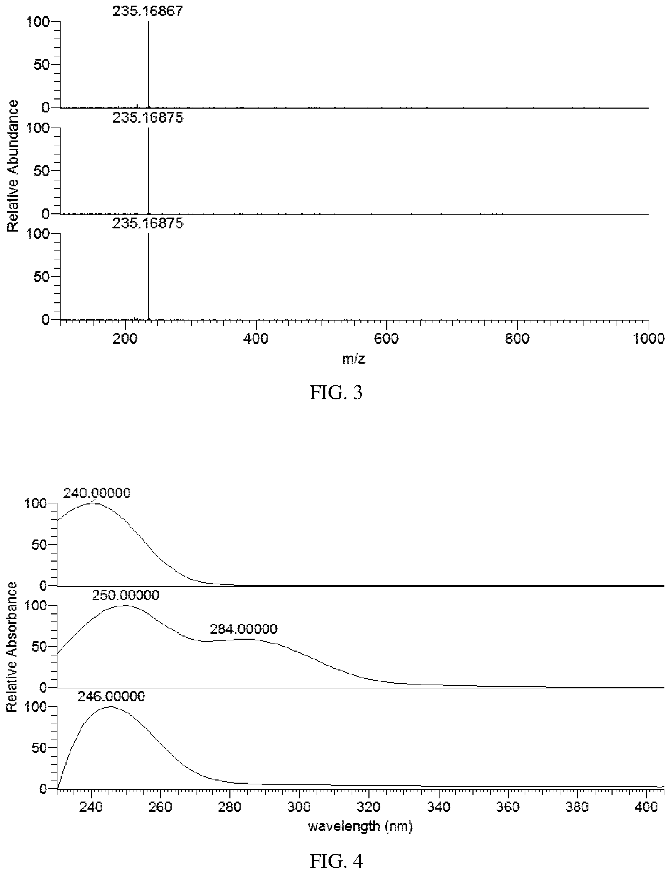

Based on the HR-MS spectra of these compounds (Fig. 3), they can be identified using the same molecular formula C15H2202 (Table 3), which refers to isomeric structures.

**Table 3. High-resolution mass-spectral (positive ion mode) data for compounds detected in Darksidea alpha culture extract.**

| Rt ^{a} | Compound Name | Formula | Detected ion | Detected formula | Calculated *m*/*z* | Found *m*/*z* | diff (ppm) |
|---|---|---|---|---|---|---|---|
| 7.3 | petasol | C₁₅H₂₂O₂ | [M+H]⁺ | C₁₅H₂₃O₂ | 235.16926 | 235.16867 | -2.494 |
| 7.6 | isopetasol | C₁₅H₂₂O₂ | [M+H]⁺ | C₁₅H₂₃O₂ | 235.16926 | 235.16875 | -2.154 |
| 8.5 | neopetasol | C₁₅H₂₂O₂ | [M+H]⁺ | C₁₅H₂₃O₂ | 235.16926 | 235.16875 | -2.154 |

| | | | | | | | |
|---|---|---|---|---|---|---|---|
| a Retention times of compounds correspond to those in Fig. 1. | | | | | | | |

The UV spectrum of compound DA-2 shows a bathochromic shift relative to the comparable UV spectra of compounds DA-1 and DA-3, suggesting a more extensive conjugated systems in compound DA-2 than in compounds DA-1 and DA-3 (Fig. 4).

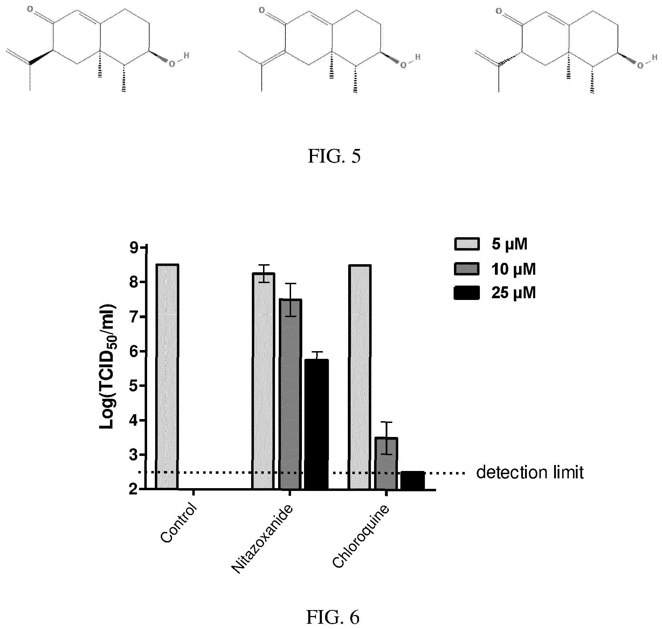

Based on these results and the NMR data of the compounds DA-1-3 identical to those reported previously for petasol isomers [Sugama, K., et al., Sesquiterpenoids from petasites fragrans. Phytochemistry, 1983. 7: 1619-22.; Neuenschwander, M., Neuenschwander, A., and Steinegger, E., Struktur der Sesquiterpene von Petasites hybridus (L.) G. M. et SCH.: Neopetasol-Abkömmlinge. Helvetica Chimica Acta, 1979.; Le, D.H., et al., Eremophilane-type sesquiterpenes from cultured lichen mycobionts of Sarcographa tricosa. Phytochemistry, 2013. 91: 242-8.], Darksidea alpha compounds DA-1, DA-2 and DA-3 were identified as petasol, isopetasol and neopetasol, respectively (Fig. 5).

The effective compound petasol is a rarely occurring natural metabolite, determined earlier only in four fungal species [Le, D.H., et al., Eremophilane-type sesquiterpenes from cultured lichen mycobionts of Sarcographa tricosa. Phytochemistry, 2013. 91: 242-8.; Bunkers, G.J. and Strobel, G.A., A proposed mode of action for green island induction by the eremophilane phytotoxins produced by Drechslera gigantea. Physiol Mol Plant Pathol, 1991. 5: 313-23.; Jayasuriya, H., et al., Identification of diverse microbial metabolites as potent inhibitors of HIV-1 Tat transactivation. Chem Biodivers, 2005. 2: 112-22.; Chen, Y., et al., Bioactive sesquiterpene derivatives from mangrove endophytic fungus Phomopsis sp. SYSU-QYP-23: Structures and nitric oxide inhibitory activities. Bioorg Chem, 2021. 107: 104530.] and two plants (i.e., Petasites formosanus and P. fragrans) [Sugama, K., et al., Sesquiterpenoids from petasites fragrans. Phytochemistry, 1983. 7: 1619-22.; Lin, Y.L., Eremophilanes from Petasits formosanus KITAMURA. Chem Pharm Bull, 1998. 46: 1807-9.]. Among these plants accumulating petasol, P. formosanus is also used as medicinal plant [Lin, Y.L., Eremophilanes from Petasits formosanus KITAMURA. Chem Pharm Bull, 1998. 46: 1807-9.] thus, suggesting the possibility of the safe use of petasol. The isolates of Darksidea alpha, a new source of the metabolites, are abundant sources of petasol, allowing the high-yield isolation of this compound through preparative HPLC. Namely, starting from 1.0 g lyophilized Darksidea alpha culture, 3.6 mg petasol could be isolated (average yield, obtained from four independently grown fungal cultures; differences could be characterized by a relative standard deviation (RSD) value of 23%). In addition to petasol, significant amount of isopetasol (1.0 mg) and neopetasol (0.33 mg) can also be isolated from 1.0 g lyophilized Darksidea alpha culture (average yields, obtained from four independently grown fungal cultures; differences could be characterized by the RSD values of 54 % (isopetasol) and 13% (neopetasol)).

### In Vitro Evaluation of the Compounds

To validate our antiviral test system, antiviral activity of two drugs (as reference compounds) were determined, namely Nitazoxanide and Chloroquine. These drugs are being studied to treat COVID-19 (Fig. 6).

Both compounds showed a concentration dependent antiviral effect on SARS-CoV-2 virus infected Vero E6 cells.

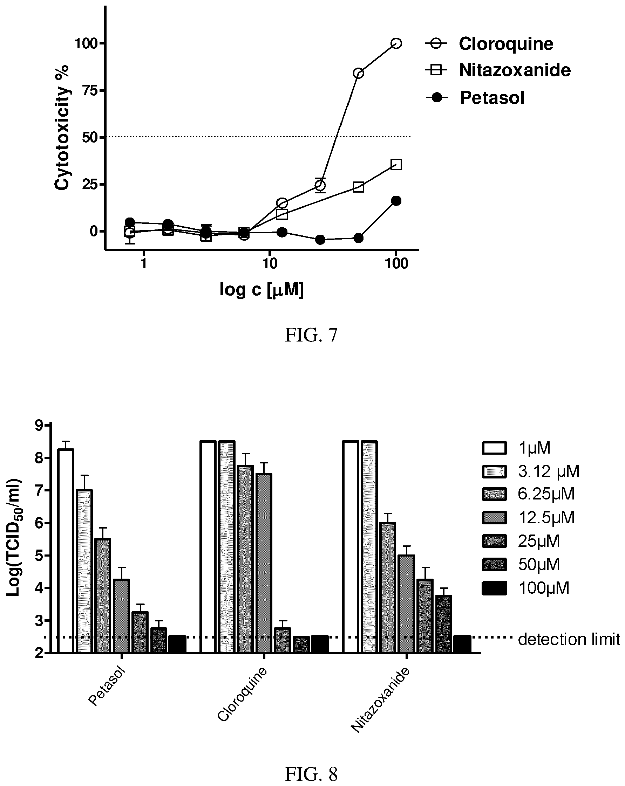

Next, *in vitro* cytotoxicity of the compounds was measured on the host cells (VeroE6). Data revealed that Petasol was not cytotoxic up to 100 µM, while Nitazoxanide showed around 35% toxicity on the host cells (Fig 7.) and the IC50 value of Chloroquine was 34 µM.

The antiviral effect of Petasol was tested on SARS-CoV-2 virus infected Vero-E6 cells. After 48 hours of incubation, the virus-induced cytopathic effect (CPE) was detected. Petasol compound showed similar antiviral effect as chloroquine and better than Nitazoxanide (Fig. 8).

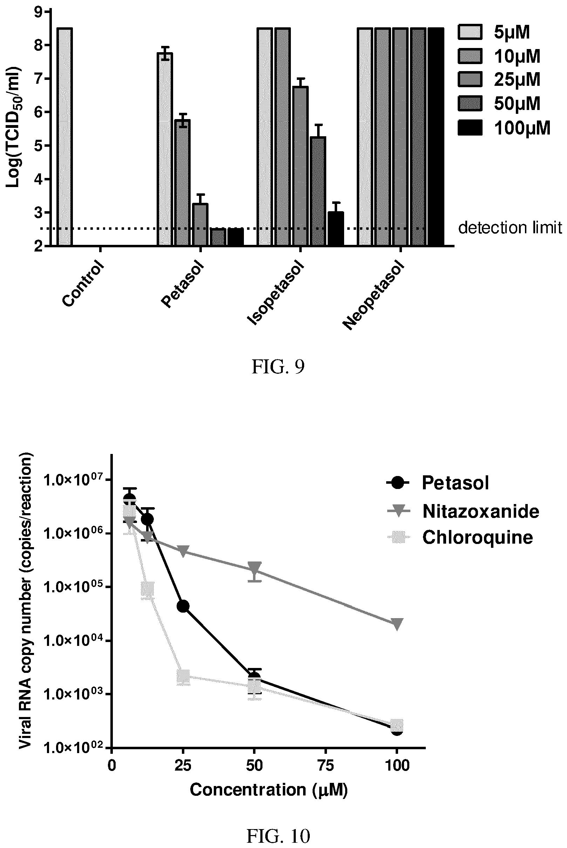

Petasol was also comparatively tested with its isomers. The antiviral activity of isopetasol and neopetasol was markedly different than petasol. This indicates that the antiviral effect is strongly dependent on the structure, namely the absolute configuration of the compound petasol (Fig. 9). Beside the virus-induced cytopathic effect (CPE), direct determination of the viral copy numbers was also assessed using a RT-qPCR method. Viral RNA copy numbers showed high similarity with the TCID50 values. Therefore, we can conclude that both read-out methods have resulted in similar antiviral effect of petasol (Fig. 10).

## Claims

1. A compound of general formula (I) for use in the treatment of a coronavirus infection, 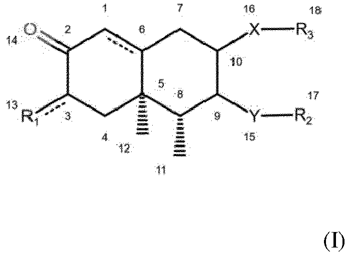 wherein in general formula (I)
the bond between carbon 1 and 6 is either a single bond or a double bond,
the bond between carbon 13 of R₁ and carbon 3 is either a single bond or a double bond,
wherein R₁ is
isopropyl, if the bond between R₁ and carbon 3 is a double bond, or isopropenyl, if the bond between R₁ and carbon 3 is a single bond,
X is O or nothing
R₂ is H or R₂ has the following formula (i) 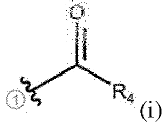
wherein in formula (i) R₄ is a C₁-C₈ alkyl or a C₁-C₈ alkenyl or a C₁-C₇ thioalkyl or a C₁-C₇ thioether, or a C₁-C₈ alkyl or a C₁-C₈ alkenyl substituted with a single -SH group, wherein said C₁-C₈ alkenyl is a straight or branched chain alkenyl;
or
X is O or nothing
R₃ is H or R₃ has the following formula (i) 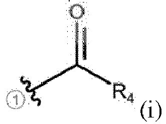
wherein in formula (i) R₄ is a C₁-C₈ alkyl or a C₁-C₈ alkenyl or a C₁-C₇ thioalkyl or a C₁-C₇ thioether, or a C₁-C₈ alkyl or a C₁-C₈ alkenyl substituted with a single -SH group, wherein said C₁-C₈ alkenyl is a straight or branched chain alkenyl;
or
Y is O or nothing
wherein one of X and Y is O, and if X is nothing, then R₃ is H and if Y is nothing then R₂ is H.

2. The compound for use according to claim 1, said compound having general formula (II) 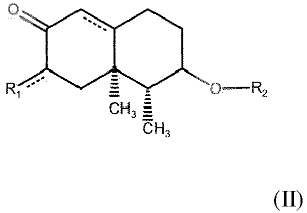 wherein in general formula (II)
the bond between carbon 1 and 6 is either a single bond or a double bond, preferably a double bond
the bond between carbon 13 of R₁ and carbon 3 is either a single bond or a double bond,
wherein R₁ is
isopropyl, if the bond between R₁ and carbon 3 is a double bond, or
isopropenyl, if the bond between R₁ and carbon 3 is a single bond,
R₂ is H or R₂ has the following formula (i) 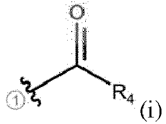 wherein in formula (i) R₄ is a C₁-C₈ alkyl or a C₁-C₈ alkenyl or a C₁-C₇ thioalkyl or a C₁-C₇ thioether, or a C₁-C₈ alkyl or a C₁-C₈ alkenyl substituted with a single -SH group, wherein said C₁-C₈ alkenyl is a straight or branched chain alkenyl.

3. The compound for use according to claim 2, wherein said compound is selected from the group consisting of compounds having general formulae IV. 1 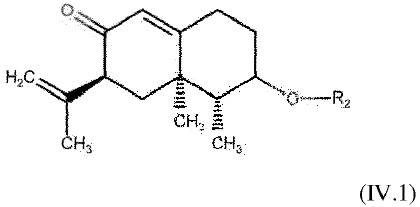 and IV.2 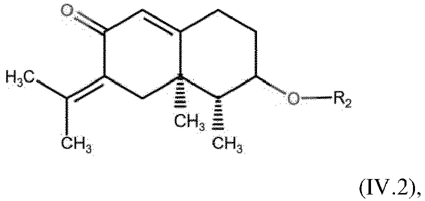 wherein R₂ is as defined in claim 2.

4. The compound for use according to any of claims 2 to 3, wherein R₂ is selected from the group consisting of

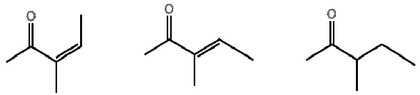

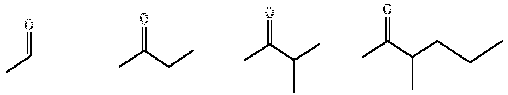

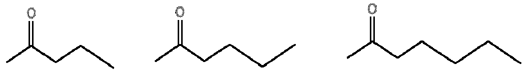

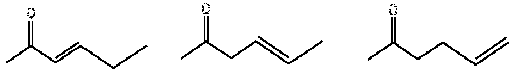

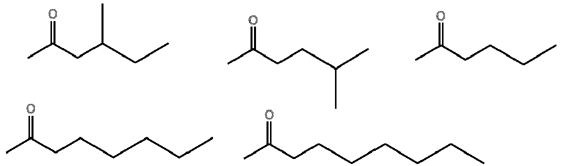

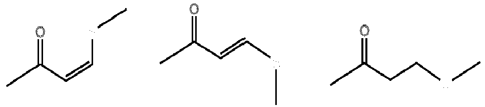

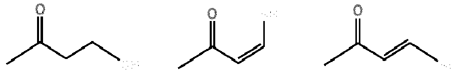

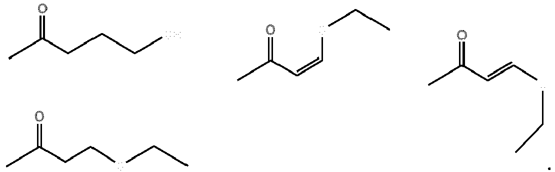

5. The compound for use according to any of the previous claims wherein said compound is selected from the group consisting of petasol and esters thereof, isopetasol and esters thereof.

6. The compound for use according to claim 5 wherein said compound is selected from the group consisting of petasol, petasin, S-petasin, isopetasol, isopetasin and S-isopetasin.

7. The compound for use according to claim 6 wherein said compound is selected from the group consisting of petasol and isopetasol.

8. The compound for use according to claim 6 wherein the compound of formula 1 is selected from the group consisting of petasol, petasin and S-petasin.

9. The compound for use according to any of the previous claims wherein the coronavirus is SARS-CoV-2.

10. A pharmaceutical composition comprising a compound as defined in any one of claims 1-8 and a pharmaceutically acceptable excipient, additive or carrier.
